(19) Europäisches Patentamt
European Patent Office
Office européen des brevets

(11) EP 2 488 291 B1

(12) EUROPÄISCHE PATENTSCHRIFT

(45) Veröffentlichungstag und Bekanntmachung des Hinweises auf die Patenterteilung:
**10.12.2014 Patentblatt 2014/50**

(21) Anmeldenummer: **10768453.2**

(22) Anmeldetag: **11.10.2010**

(51) Int Cl.:
***B01J 2/04*** (2006.01) ***C07C 227/42*** (2006.01)
***C11D 3/33*** (2006.01) ***C11D 11/00*** (2006.01)
***C07C 229/24*** (2006.01) ***C11D 17/06*** (2006.01)

(86) Internationale Anmeldenummer:
**PCT/EP2010/065184**

(87) Internationale Veröffentlichungsnummer:
**WO 2011/045266 (21.04.2011 Gazette 2011/16)**

(54) **VERFAHREN ZUR HERSTELLUNG EINES PULVERS ENTHALTEND EINES ODER MEHRERE KOMPLEXBILDNERSALZE**

METHOD FOR PRODUCING A POWDER CONTAINING ONE OR MORE COMPLEXING AGENT SALTS

PROCÉDÉ DE FABRICATION D'UNE POUDRE CONTENANT UN OU PLUSIEURS SELS COMPLEXANTS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **12.10.2009 EP 09172810**

(43) Veröffentlichungstag der Anmeldung:
**22.08.2012 Patentblatt 2012/34**

(73) Patentinhaber: **BASF SE**
**67056 Ludwigshafen (DE)**

(72) Erfinder:
- **MRZENA, Frank**
  **67112 Mutterstadt (DE)**
- **MENGE, Ulrich**
  **79639 Grenzach-Wyhlen (DE)**
- **SCHÖNHERR, Michael**
  **67227 Frankenthal (DE)**
- **HEIDENFELDER, Thomas**
  **69493 Hirschberg (DE)**

(56) Entgegenhaltungen:
**DE-A1- 2 125 945** **DE-B- 1 261 619**
**US-A- 2 970 057** **US-A- 3 615 723**
**US-A- 4 070 766** **US-A1- 2002 046 427**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 488 291 B1

## Beschreibung

Die Erfindung betrifft ein Verfahren zur Herstellung eines Pulvers enthaltend eines oder mehrere Komplexbildnersalze der allgemeinen Formel I

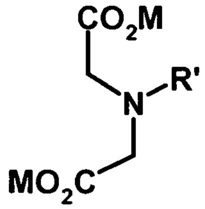

(I)

sowie eine Verwendung der obigen Pulver.

Die häufig als Komplexbildner beispielsweise in Wasch- und Reinigungsmitteln eingesetzten Aminopolyphosphonate, Polycarboxylate oder Aminopolycarboxylate, wie Ethylendiamintetraessigsäure (EDTA), sind nur in geringem Maße biologisch abbaubar.

Eine preiswerte Alternative stellen die Glycin-N,N-diessigsäurederivate, wie die Methylglycin-N,N-diessigsäure (MGDA) und deren Salze - z.B. die Trialkalimetallsalze - dar, welche vorteilhafte toxikologische Eigenschaften aufweisen und gut biologisch abbaubar sind. Die Verwendung von MGDA und von verwandten Glycin-N,N-diessigsäurederivaten in Reinigungsmitteln sowie deren Synthesen sind z.B. in WO-A 94/029421 oder US 5,849,950 beschrieben. Für eine kostengünstige Produktion der Glycin-N,N-diessigsäurederivate werden hohe Anforderungen an die Ausbeute der einzelnen Syntheseschritte und Reinheit der isolierten Zwischenprodukte gestellt.

MGDA wird insbesondere durch Umsetzung von Iminodiacetontril mit Acetaldehyd und Blausäure oder von alpha-Alaninnitril mit Formaldehyd und Blausäure und alkalischer Hydrolyse des als Zwischenprodukt erhaltenen Methylglycindiacetonitrils (MGDN) mit Natronlauge hergestellt, wobei das Trinatriumsalz des MGDA erhalten wird. Um hohe MGDA-Ausbeuten und -Reinheiten zu erzielen, wird MGDN in der Regel als Zwischenprodukt isoliert und als Reinstoff in dem sich anschließenden Hydrolyseschritt eingesetzt.

Problematisch bei der Hydrolyse von Alkylglycinnitril-N,N-diacetonitrilen ist ihre thermische Labilität, insbesondere im alkalischen Milieu. Durch die sterisch anspruchsvolle Alkylsubstitution werden Rückspaltungsreaktionen begünstigt. Deshalb sind Verfahren entwickelt worden, die möglichst zu nebenproduktarmen Formen des MGDA und seiner Salze führen.

Ein verbessertes Verfahren zur Herstellung von nebenproduktarmen Salzen des MGDA wird in der WO 2006/120129 beschrieben. Die moderneren Herstellverfahren führen im Allgemeinen zu etwa 35-40 gew.-%igen wässrigen Lösungen, aus denen danach die Salze in fließfähiger Form hergestellt werden.

Eines der bekannten Aufarbeitungsverfahren im Stand der Technik ist die Überführung derartiger wässriger Lösungen in einen Sprühturm. Dabei entstehen überwiegend amorphe Pulver mit einer Restfeuchte in der Größenordnung von beispielsweise 5 Gew.-%. Höhere Restfeuchten sind zwar denkbar, aber in einem Sprühturm eher schwierig zu erzeugen und darüber hinaus auch unerwünscht, weil es dann bei der späteren Lagerung beim Abnehmer oder bei der Verarbeitung Verklumpungen der Pulver geben kann. Es ist weiterhin bekannt, dass ein Granulat solche Nachteile nicht hat und sich deshalb problemloser verarbeiten lässt. Allerdings verlangt die Granulaterzeugung einen zusätzlichen Umarbeitungsschritt nach der Pulvererzeugung im Sprühturm und ist deshalb relativ teuer. Bei diesem Umarbeitungsschritt wird dem Pulver aus dem Sprühturm zusätzliche Feuchtigkeit zugeführt, und es wird unter Erwärmen und Kneten bei einer Verweilzeit in der Größenordnung einer Stunde über eine Kristallisation granuliert. Ein derartiges Verfahren ist beispielsweise in EP-A 08 45 456 beschrieben.

Es war demgegenüber Aufgabe der Erfindung, ein technisch einfaches Verfahren zur Herstellung von Pulvern der obigen Komplexbildnersalze zur Verfügung zu stellen, die die für den weiteren Einsatz derselben gewünschten Eigenschaften aufweisen, insbesondere gute Lager- und Verarbeitungseigenschaften, die einen erhöhten Kristallinitätsgrad und eine erhöhte Restfeuchte im Bereich von etwa 7 bis 14 Gew.-%, bezogen auf das Gesamtgewicht des Pulvers, aufweisen, und granulatförmig sind.

Die Aufgabe wird gelöst durch ein Verfahren zur Herstellung eines Pulvers enthaltend eines oder mehrere Komplexbildnersalze der allgemeinen Formel

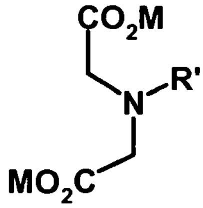

**(I)**

in der

R' Wasserstoff oder eine der Gruppierungen

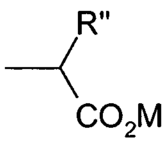

oder

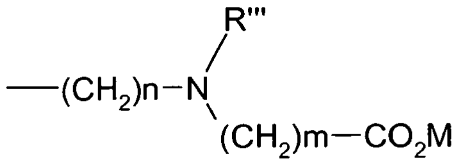

bedeutet, wobei

R" Wasserstoff, ein $C_1$-$C_{12}$-Alkylrest oder ein -$(CH_2)_q$-COOM-Rest mit q = 1 bis 5 ist
n und m jeweils eine ganze Zahl von 0 bis 5 sind und
R'" Wasserstoff oder ein $C_1$-$C_{12}$-Alkylrest oder ein $C_2$-$C_{12}$-Alkenylrest ist, der zusätzlich mit bis zu 5 Hydroxylgruppen substituiert sein kann, oder eine der Gruppierungen

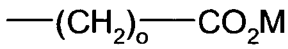

oder

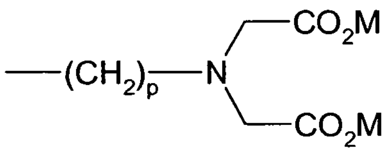

ist, in der o und p jeweils eine ganze Zahl von 0 bis 5 sind, und M unabhängig voneinander Wasserstoff, Alkalimetall, Erdalkalimetall, Ammonium oder substituiertes Ammonium in den entsprechenden stöchiometrischen Mengen bedeutet,

- ausgehend von einer wässrigen Lösung, enthaltend das eine oder die mehreren Komplexbildnersalze in einer Konzentration von 10 bis 80 Gew.-%, bezogen auf das Gesamtgewicht der wässrigen Lösung,
- in einem Sprühtrocknungsverfahren, umfassend einen Zerstäubungsschritt und einen Trocknungsschritt,

das dadurch gekennzeichnet ist, dass
der Zerstäubungsschritt unter Zusatz von kristallinem Feinstaub desselben oder derselben Komplexbildnersalze, wie sie in der wässrigen Lösung enthalten sind, oder eines oder mehrerer hiervon verschiedener Komplexbildnersalze der obigen Formel I, mit einer Obergrenze für den mittleren Partikeldurchmesser des kristallinen Feinstaubs, die um mindestens den Faktor 2 kleiner ist als die Untergrenze des mittleren Partikeldurchmessers des nach dem Verfahren erhaltenen Pulvers,
in einem Anteil von 0,1 bis 20 Gew.-%, bezogen auf das Gewicht des nach dem Verfahren erhaltenen Pulvers, durchgeführt wird.

Es wurde gefunden, dass es möglich ist, in einem einfachen Sprühtrocknungsverfahren Pulver der obigen Komplexbildnersalze zu erhalten, die die zur Lagerung und Verarbeitung erwünschten Eigenschaften aufweisen, indem von einer wässrigen Lösung derselben ausgegangen wird, die in Gegenwart eines kristallinen Feinstaubes zerstäubt wird, das wesentlich kleinere Partikeldimensionen aufweist als die Untergrenze des mittleren Partikeldurchmessers des nach dem Verfahren erhaltenen Pulvers.

Für das erfindungsgemäße Verfahren ist es wesentlich, dass im Zerstäubungsschritt ein Spray zur Verfügung gestellt wird, das in einer kontinuierlichen Phase eines Inertgases, insbesondere Luft, als disperse Phase Flüssigkeitströpfchen der wässrigen Ausgangslösung, enthaltend das eine oder die mehreren Komplexbildnersalze und daneben eine weitere disperse feste Phase, enthaltend den kristallinen Feinstaub eines oder mehrerer Komplexbildnersalze, enthält. Es wird angenommen, dass der kristalline Feinstaub Kristallisationskeime während des Trocknungsprozesses anbietet, an denen die Tröpfchen der wässrigen Lösung auskristallisieren und aufwachsen können. Darüber hinaus

kann durch Rückführung des feinen, getrockneten Sprühpulvers in die Trocknungszone die Partikelgröße in derselben erhöht und die Granulometrie des im Verfahren erhaltenen Pulvers positiv beeinflusst werden. Das kristalline Feinpulver hat darüber hinaus auch die Wirkung einer Abpuderung des in der Sprühtrocknungsanlage erhaltenen feuchten Pulvers.

Im Rahmen der vorliegenden Erfindung wird unter dem Begriff Pulver ein fließfähiger Feststoff verstanden, der mittlere Partikelgrößen im Bereich von ca. 1 $\mu$m bis ca. 10 mm aufweist; für die gröberen Partikel aus dem vorstehend definierten Bereich, ab ca. 100 $\mu$m, ist alternativ auch die Bezeichnung "Granulat" gebräuchlich.

Das Pulver enthaltend eines oder mehrere der obigen Komplexbildnersalze wird erfindungsgemäß in einem Sprühtrocknungsverfahren hergestellt.

Die historisch gewachsenen fachspezifischen Begriffe auf dem Gebiet der Sprühtrocknung werden häufig nicht einheitlich angewandt; daher sollen im Folgenden die für die vorliegende Erfindung maßgeblichen Begriffe erläutert werden:

Der Begriff Sprühtrocknungsverfahren bezeichnet vorliegend als Oberbegriff sämtliche Verfahren, bei denen ein flüssiger Ausgangsstoff, der als Lösung oder Dispersion vorliegen kann, mit dem Ziel der Herstellung eines Feststoffes zerstäubt und getrocknet wird. Ein Sprühtrocknungsverfahren ist durch die Verfahrensschritte Zerstäubung und Trocknung gekennzeichnet. Diese können in demselben Apparat oder auch in hintereinandergeschalteten Bereichen desselben Apparates durchgeführt werden.

Der erste Verfahrensschritt jedes Sprühtrocknungsverfahrens ist die Zerstäubung des flüssigen Ausgangsstoffes in ein Inertgas, in der Regel Luft, unter Erhalt eines Sprays, wobei das Spray als kontinuierliche Phase das Inertgas, in der Regel Luft, und als diskontinuierliche Phasen feinverteilte Flüssigkeitströpfchen des flüssigen Ausgangsstoffes und darüber hinaus zusätzlich, entsprechend dem Verfahren der Erfindung, eine disperse feste Phase umfasst, gebildet aus dem kristallinen Feinstaub des einen oder der mehreren Komplexbildnersalze. Das Spray wird durch eine bestimmte Tröpfchengrößenverteilung und Tröpfchengrößenverteilungsbreite (Span) charakterisiert.

Unter den Oberbegriff Sprühtrocknungsverfahren werden insbesondere die nachfolgend aufgeführten besonderen Verfahren subsumiert:

Die Sprühtrocknung im engeren Sinne, die agglomerierende Sprühtrocknung, die Sprühagglomeration sowie die Sprühgranulation.

Die Sprühtrocknung im engeren Sinne ist das historisch älteste Sprühtrocknungsverfahren. Sie wird in Sprühtürmen durchgeführt. Die ersten Sprühtürme wurden in den 1930er Jahren von der Firma Niro gebaut. Obwohl die Bauarten von Sprühtürmen unterschiedlich sind, ist das Prinzip stets das gleiche: Aus jedem Tröpfchen des im Sprühturm erhaltenen Sprays soll jeweils genau ein Partikel entstehen. Da die Verweilzeit in den Sprühtürmen begrenzt ist, dürfen die Tröpfchen im Sprühturm nur sehr klein sein, um im Sprühturm trocknen zu können. Die mittlere Partikelgröße von Sprühpulvern, die durch Sprühtrocknung im engeren Sinne erhalten wird, liegt häufig im Bereich von ca. 50 $\mu$m bis etwa 300 $\mu$m.

Nach einer bevorzugten Ausführungsform der Sprühtrocknung in Sprühtürmen kann eine so genannte agglomerierende Sprühtrocknung durchgeführt werden, die von Sprühturmherstellern in den 1980er Jahren entwickelt wurde, um den Staubanteil im Sprühpulver zu reduzieren. Bei diesem Verfahren wird der Feinanteil des Sprühpulvers im Sprühturm separiert und in den Bereich des Zerstäubers rezykliert. Dort kommen die Partikeln mit dem noch flüssigen Spray in Kontakt und können agglomerieren, d.h. zwei oder mehrere kleine Teilchen verbinden sich zu jeweils einem größeren Teilchen, dem so genannten Agglomerat. Da die Agglomerate längere Trocknungszeiten benötigen gegenüber Einzelpartikeln, wird in den Sprühturm zwecks Erhöhung der Verweilzeit eine Wirbelschicht integriert. Derartige Sprühtürme mit integrierter Wirbelschicht sind beispielsweise als Fluidized Spray Dryer (FSD) der Firma Niro oder Spray Bed Dryer (SBD) der Firma Anhydro bekannt.

In einer weiteren Variante eines Sprühtrocknungsverfahrens, der so genannten Sprühagglomeration, wird Pulver, das in einem Mischer mit bewegten Einbauten vorliegt, durch Einsprühen von Bindeflüssigkeit zu größeren Teilchen, so genannten Agglomeraten, verbunden. Bei der Sprühagglomeration in einem Mischer muss ein Trockner nachgeschaltet werden, der insbesondere eine Wirbelschicht sein kann. Die Sprühagglomeration kann sowohl kontinuierlich als auch diskontinuierlich durchgeführt werden.

In einer weiteren Verfahrensvariante der Sprühtrocknungsverfahren, der so genannten Sprühgranulation, wird der flüssige Ausgangsstoff in eine Wirbelschicht eingesprüht. Die Tropfen des Sprays werden dabei überwiegend auf den in der Wirbelschicht bereits vorhandenen Granulaten abgeschieden und tragen zu deren weiterem Wachstum bei. Das Endprodukt wird häufig über Sieben oder Sichten aus der breiteren Wirbelschicht-Bettfraktion gewonnen. Grobgut wird oft gemahlen und gemeinsam mit dem abgetrennten Feingut in die Wirbelschicht zurückgeführt. Der Prozess ist damit komplizierter als die Sprühtrocknung im engeren Sinne oder die Sprühagglomeration. Es können jedoch größere Partikeln und engere Partikelgrößenverteilungen erzielt werden.

Das erfindungsgemäße Sprühtrocknungsverfahren kann bevorzugt in jeder der oben geschriebenen Verfahrensvarianten durchgeführt werden, wobei jeweils zwingend ist, dass im Zerstäubungsschritt kristalliner Feinstaub desselben oder derselben Komplexbildnersalze wie sie in der wässrigen Ausgangslösung enthalten sind, oder eines oder mehrere hiervon verschiedene Komplexbildnersalze der obigen Formel I eingesetzt werden, mit einer Obergrenze für den mittleren Partikeldurchmesser des kristallinen Feinstaubs, der um mindestens den Faktor 2 kleiner ist als die Untergrenze des mittleren Partikeldurchmessers des nach dem Sprühtrocknungsverfahren erhaltenen Pulvers.

Die mittleren Partikeldurchmesser des kristallinen Feinstaubs sowie des nach dem erfindungsgemäßen Verfahren erhaltenen Pulvers werden üblicherweise nach Methoden wie Laserbeugung (z.B. Malvern) oder optischen Verfahren (z.B. CamSizer) bestimmt.

Wenn als Sprühtrocknungsverfahren eine Sprühtrocknung im engeren Sinne durchgeführt wird, liegen die mittleren Partikeldurchmesser der hiernach erhaltenen Pulver häufig in einem Bereich von ca. 50 bis 300 μm. Entsprechend muss im Zerstäubungsschritt kristalliner Feinstaub mit einer Obergrenze für den mittleren Partikeldurchmesser von maximal 25 μm eingesetzt werden.

Wird als Sprühtrocknungsverfahren eine Sprühgranulation durchgeführt, so werden Pulver erhalten, die häufig einen mittleren Partikeldurchmesser im Bereich von etwa 200 bis 2000 μm aufweisen. Entsprechend muss kristalliner Feinstaub eingesetzt werden, für den die zulässige Obergrenze für den mittleren Partikeldurchmesser maximal 100 μm beträgt.

Der Gewichtsanteil für den Zusatz an kristallinem Feinstaub im Sprühtrocknungsverfahren liegt erfindungsgemäß im Bereich von etwa 0,1 bis 20 Gew.-%, bezogen auf das Gewicht des nach dem Verfahren erhaltenen Pulvers, bevorzugt etwa 4 bis 10 Gew.-%, bezogen auf das Gesamtgewicht des nach dem Verfahren erhaltenen Pulvers.

Bevorzugt wird von einer wässrigen Lösung, die nach der entsprechenden Synthese erhalten wird, ausgegangen, die ca. 30 bis 50 Gew.-% des einen oder der mehreren Komplexbildnersalze enthält, und die in einem dem Sprühtrocknungsverfahren vorgeschalteten Verfahrensschritt in einem Wärmetauscher oder einem Dünnschichtverdampfer auf ca. 55 bis 80 Gew.-% an Komplexbildnersalzen, bezogen auf das Gesamtgewicht der wässrigen Lösung, aufkonzentriert wird.

Das eine oder die mehreren Komplexbildnersalze entsprechen der allgemeinen Formel

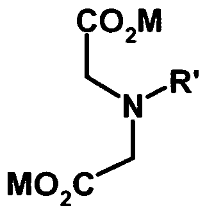

**(I)**

in der

R' Wasserstoff oder eine der Gruppierungen

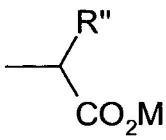

oder

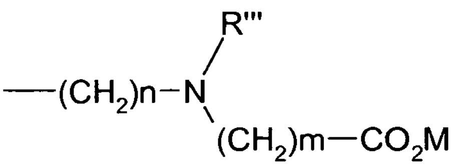

bedeutet, wobei

R" Wasserstoff, ein $C_1$-$C_{12}$-Alkylrest oder ein $-(CH_2)_q$-COOM-Rest mit q = 1 bis 5 ist

n und m jeweils eine ganze Zahl von 0 bis 5 sind und

R"' Wasserstoff oder ein $C_1$-$C_{12}$-Alkylrest oder ein $C_2$-$C_{12}$-Alkenylrest ist, der zusätzlich mit bis zu 5 Hydroxylgruppen substituiert sein kann, oder eine der Gruppierungen

$-(CH_2)_o-CO_2M$ oder

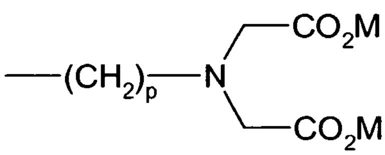

ist, in der o und p jeweils eine ganze Zahl von 0 bis 5 sind, und
M unabhängig voneinander Wasserstoff, Alkalimetall, Erdalkalimetall, Ammonium oder substituiertes Ammonium in den entsprechenden stöchiometrischen Mengen bedeutet.

Bevorzugt handelt es sich hierbei um Derivate der Glycin-N,N-Diessigsäure oder Derivate der Glutamin-N,N-Diessigsäure. Bevorzugt sind auch Derivate der Ethylendiamintriessigsäure oder der Nitrilotriessigsäure.

Besonders bevorzugt sind als Derivate der Glycin-N,N-Diessigsäure Alkalisalze der Methylglycin-N,N-diessigsäure, im Folgenden als MGDA bezeichnet.

Der Trocknungsschritt des Sprühtrocknungsverfahrens wird bevorzugt bei einem Druck im Bereich von etwa 0,1 bar absolut bis 10 bar absolut, insbesondere bei einem Druck im Bereich von etwa 0,8 bar absolut bis 2 bar absolut, durchgeführt.

Die Verweilzeit im Trocknungsschritt liegt vorzugsweise in einem Bereich von etwa 10 Sekunden bis zu 1 h.

Gegenstand der Erfindung ist auch eine Formulierung, enthaltend das gemäß dem vorstehend beschriebenen Verfahren erhaltene Pulver oder eine wässrige Lösung desselben als Komplexbildner für Erdalkali- und Schwermetallionen, in den hierfür üblichen Mengen, neben anderen üblichen Bestandteilen solcher Formulierungen.

Die Formulierungen können insbesondere Wasch- und Reinigungsmittelformulierungen sein.

Ein weiterer Gegenstand der Erfindung ist auch die Verwendung eines nach dem obigen Verfahren erhaltenen Pulvers zur Herstellung von Pressagglomeraten, sowie eine Verwendung der Pressagglomerate zum Einsatz in festen Reinigungsmitteln.

Die obigen Reinigungsmittel können insbesondere für automatische Geschirrspülmaschinen vorgesehen sein. Insbesondere kann es sich hierbei um Tabs für Geschirrspülmaschinen handeln.

Das erfindungsgemäße Sprühtrocknungsverfahren kann auch mit Mischungen aus einem oder mehreren Komplexbildnersalzen und weiteren Stoffen durchgeführt werden. Unter weiteren Stoffen sind insbesondere üblicherweise in der Wasch- und Reinigungsmittelindustrie eingesetzte Hilfs- und Zusatzstoffe zu verstehen. Beispielsweise können Tenside, Polymere, anorganische Salze, und/oder Zitrate eingesetzt werden. Für den Einsatz im Bereich der maschinellen Geschirrreinigung bieten sich beispielsweise anorganische Salze, wie Carbonate, Sulfate, Phosphate, Silikate; organische Salze wie Zitrate; Polymere, wie Polycarboxylate oder sulfonierte Polymere oder Phosphonate an. Durch derartige Mischungen lässt sich der Produktionsprozess für die Herstellung von Wasch- und Reinigungsmitteln einfacher gestalten.

Die nach dem obigen Verfahren erhaltenen Pulver können insbesondere auch in Abmischungen mit üblichen Hilfs- und Zusatzstoffen eingesetzt werden.

Nach dem erfindungsgemäßen Verfahren kann insbesondere ein Methylglycin-N,N-Diessigsäure-Trinatriumsalzpulver mit einem Kristallinitätsgrad von ≥30 % enthaltend eine erste kristalline Modifikation mit den nachfolgend angegebenen d-Werten in Angström bei den Beugungswinkeln 2-theta in ° erhalten werden:

| 2-theta (°) | d Wert (Angström) |
|---|---|
| 8.4 | 10.5 |
| 9.5 | 9.3 |
| 11.1 | 8.0 |
| 13.2 | 6.7 |
| 13.9 | 6.35 |
| 15.8 | 5.6 |
| 16.5 | 5.36 |
| 16.84 | 5.26 |
| 17.34 | 5.11 |
| 17.67 | 5.02 |
| 18.92 | 4.69 |
| 20.29 | 4.37 |
| 21.71 | 4.09 |
| 22.3 | 3.98 |
| 23.09 | 3.85 |

(fortgesetzt)

| 2-theta (°) | d Wert (Angström) |
|---|---|
| 24.74 | 3.59 |
| 25.36 | 3.51 |
| 27.04 | 3.29 |
| 28.28 | 3.15 |
| 29.63 | 3.01 |
| 30.09 | 2.97 |

und/oder eine zweite kristalline Modifikation, mit den d-Werten in Angström bei den jeweiligen Beugungswinkeln 2-theta in ° im Röntgen-Pulver-Diffraktogramm entsprechend der nachfolgenden Tabelle:

| 2-theta (°) | d Wert (Angström) |
|---|---|
| 8.2 | 10.80 |
| 10.5 | 8.40 |
| 15.55 | 5.70 |
| 16.47 | 5.38 |
| 17.09 | 5.18 |
| 18.10 | 4.90 |
| 18.82 | 4.71 |
| 21.00 | 4.23 |
| 21.35 | 4.16 |
| 22.64 | 3.92 |
| 23.69 | 3.75 |
| 24.73 | 3.60 |
| 26.75 | 3.33 |
| 28.93 | 3.08 |
| 29.88 | 2.99 |
| 31.46 | 2.84 |
| 31.88 | 2.80 |

Die Erfindung wird im Folgenden anhand einer Zeichnung sowie von Ausführungsbeispielen näher erläutert.
In der Zeichnung zeigen im Einzelnen:

| | |
|---|---|
| Figur 1 | ein Röntgendiffraktogramm für das nach Ausführungsbeispiel 1 (zum Vergleich) erhaltene Pulver, |
| die Figuren 2 bis 4 | Röntgendiffraktogramme für jeweils nach den Ausführungsbeispielen 2 bis 4 (erfindungsgemäß) erhaltene Pulver. |

Hierbei ist in den Figuren auf der Abszisse der Beugungswinkel 2-theta in °, und auf der Ordinate die gemessene Intensität, in Counts (Impulse) (dimensionslos) aufgeführt.
Die Röntgenpulverdiffraktometer-Messungen wurden an einem Diffraktometer D8 Advance® der Fa. Bruker AXS (Karlsruhe) durchgeführt. Gemessen wurde in Reflexion mit Cu-K $\alpha$-Strahlung mit einer variablen Blendeneinstellung primär- und sekundärseitig. Der Messbereich betrug 2° bis 80° 2-theta, die Schrittweite 0,01° und die Messzeit pro Winkelschritt 3,6 Sekunden.

Aus den Röntgenpulverdiffraktogrammen wurde der Kristallinitätsgrad in bekannter Weise ermittelt, indem, wie üblich, der Flächenanteil der kristallinen Phase und der amorphen Phase bestimmt wurde und hieraus der Kristallinitätsgrad, KG, als das Verhältnis der Fläche der kristallinen Phase, $I_c$, zur Gesamtfläche, bestehend aus der Fläche der amorphen Phase, $I_a$, und der Fläche der kristallinen Phase, $I_c$, berechnet:

$$KG = I_c/(I_c+I_a).$$

Die Bestimmung des Kristallinitätsgrads kann insbesondere mit einem Softwareprogramm, beispielsweise dem Softwareprogramm TOPAS® der Fa. Bruker AXS durchgeführt werden.

Hierzu wird zunächst eine amorphe Probe vermessen und der Linienverlauf mit Hilfe von sechs Einzellinien in einem Profilfit angefittet. Danach werden die Linienlagen dieser Linien sowie ihre Halbwertsbreiten fixiert und diese Werte als "amorphe Phase" abgespeichert.

Bei der zu vermessenden Probe, für die der Kristallinitätsgrad bestimmt werden soll, wird nun der Flächenanteil der kristallinen Phase und der Flächenanteil der amorphen Phase bestimmt und hieraus nach der vorstehend angegebenen Formel der Kristallinitätsgrad KG berechnet.

Die amorphe Phase wird wie oben definiert verwendet.

Die kristalline Phase kann ebenfalls über ihre Einzellinienlagen analog zur amorphen Phase definiert werden, oder anhand folgender Gitterkonstanten, als sog. (hkl)-Phase (a = 33,63, b = 11,36 und c = 6,20 und Raumgruppe Pbcm), wobei die Gitterparameter frei verfeinerbare Variablen sind. Der Untergrund wird als Polynom 1. Grades angefittet.

Das Programm TOPAS® berechnet die optimale Anpassung zwischen gemessenem Diffraktogramm und dem theoretischen Diffraktogramm bestehend aus amorpher und kristalliner Phase.

Ausführungsbeispiele

<u>Ausführungsbeispiel 1 (zum Vergleich) klassische Sprühtrocknung ohne Zusatz von kristallinem Feinstaub</u>

Ein Mengenstrom von 60 kg/h einer wässrigen Lösung von Na3-MGDA mit einem Feststoffgehalt von 40% wurde in einem Plattenwärmetauscher-Verdampfer (Heizfläche 1,7 $m^2$) auf einen Feststoffgehalt von 59% eingedampft und in einem Abscheidebehälter abgeschieden. Die Eindampfung erfolgte bei einer Wandtemperatur von 152°C (Dampfbeheizung) und bei einem Druck von 2,5 bar abs im Abscheider. Die eingedampfte Lösung wurde bei einer Temperatur von ca. 128°C mit einer Zahnradpumpe in die nachgeschaltete Kolbenmembranpumpe dosiert und mit einer Einstoffdüse in einem Sprühturm versprüht.

Der Sprühturm hatte einen Durchmesser von 800 mm und eine Länge von 12 m. Der Sprühturm wurde mit einer Luftmenge von 1400 kg/h und einer Gaseintrittstemperatur von 160°C betrieben. Die Produktaustrittstemperatur betrug 127°C und der Feststoffgehalt des Trockenproduktes 94,1%. Das Produkt wurde über einen 2-Punktaustrag (direkt am Sprühturm und am nachgeschalteten Filter) abgeschieden.

Das so hergestellte Produkt war ein rieselfähiges Pulver. Das Schüttgewicht betrug 529 kg/$m^3$. Die Röntgenstrukturanalyse zeigt, dass das Produkt amorph ist.

Das Lagerverhalten dieser Probe wurde im Exsikkator-Test bewertet. Dazu wird eine Probe von 3 g in einem offenen Wägeschälchen in einen Exsikkator bei 20°C und einer relativen Luftfeuchte von 76% über einen Zeitraum von 144 Stunden gelagert. Anschließend wird die Massezunahme der Probe ermittelt und die Rieselfähigkeit der Probe bewertet. Die Massezunahme betrug 27,1% und die Probe war angelöst, d.h. sie war nass und nicht mehr rieselfähig.

<u>Ausführungsbeispiel 2 (erfindungsgemäß) Sprühturm mit Zugabe von kristallinem Feinstaub</u>

Ein Mengenstrom von 75 kg/h einer wässrigen Lösung von Na3-MGDA mit einem Feststoffgehalt von 40% wurde in einem Plattenwärmetauscher-Verdampfer (Heizfläche 1,7 $m^2$) auf einen Feststoffgehalt von 60% eingedampft und in einem Abscheidebehälter abgeschieden. Die Eindampfung erfolgte bei einer Wandtemperatur von 156°C (Dampfbeheizung) und bei einem Druck von 2,5 bar abs im Abscheider. Die eingedampfte Lösung wurde bei einer Temperatur von ca. 130°C mit einer Zahnradpumpe in die nachgeschaltete Kolbenmembranpumpe dosiert und mit einer Einstoffdüse in einem Sprühturm versprüht.

Der Sprühturm hatte einen Durchmesser von 800 mm und eine Länge von 12 m. Der Sprühturm wurde mit einer Luftmenge von 1400 kg/h und einer Gaseintrittstemperatur von 202°C betrieben. In den Sprühturm wurde mittels eines Injektors ein Massenstrom von 4 kg/h kristalliner Feinstaub Na3-MGDA eingeblasen. Die Produktaustrittstemperatur betrug 99°C und der Feststoffgehalt des Trockenproduktes 90,2%. Das Produkt wurde über einen 2-Punktaustrag (direkt am Sprühturm und am nachgeschalteten Filter) abgeschieden.

Das so hergestellte Produkt war ein rieselfähiges Pulver. Das Schüttgewicht betrug 568 kg/$m^3$. Die Röntgenstrukturanalyse zeigt, dass das Produkt kristallin ist.

Das Lagerverhalten dieser Probe wurde im Exsikkator-Test bewertet. Dazu wird eine Probe von 3 g in einem offenen Wägeschälchen in einen Exsikkator bei 20°C und einer relativen Luftfeuchte von 76% über einen Zeitraum von 144 Stunden gelagert. Anschließend wird die Massezunahme der Probe ermittelt und die Rieselfähigkeit der Probe bewertet. Die Massezunahme betrug 20,4% und die Probe war nur gering verbacken und ließ sich durch leichtes Klopfen wieder in den rieselfähigen Zustand bringen.

<u>Ausführungsbeispiel 3 (erfindungsgemäß) agglomerierende Sprühtrocknung in einem Sprühturm mit integrierter Wirbelschicht (Fluidized Spray Dryer (FSD))</u>

500 g einer 41 %igen wässrigen Na3-MGDA-Lösung mit einem Gesamt-Feststoffgehalt von 46 Gew.-%, wurde mit 150 g deionisiertem Wasser verdünnt. Die Lösung wurde anschließend in einem Glaskolben mit Rührer bei Raumtemperatur gerührt und danach in einem Sprühtrockner mit integrierter Wirbelschicht im Labormaßstab zugeführt, unter Zuführung von Trocknungsluft bei 130°C, einer Zuluft-Temperatur der Wirbelschicht von 110°C und über eine Zweistoffdüse zerstäubt. In der ersten Phase des Trocknungsprozesses wurden die Flüssigkeitströpfchen getrocknet, unter Ausbildung der Granulationskeime im Bett. Die Betttemperatur wurde sodann abgesenkt, um die Granulationsphase zu initiieren, wobei die Granulationskerne mit Feedlösung agglomeriert wurden. Das erhaltene Granulat wurde kontinuierlich aus dem Sprühtrockner entnommen. Die Granulation der Lösung wurde in einem Bereich für die Betttemperatur zwischen 64°C und 74°C durchgeführt. Das Produkt hatte eine Restfeuchte von 6,5 Gew.-%, eine hohe Schüttdichte von 700 kg/$m^3$ und war sehr gut rieselfähig. Nach 144 Stunden im Eksikkator bei 20°C und 76 % relative Feuchtigkeit blieb das Produkt rieselfähig, das Röntgendiffraktogramm (Figur 3) weist einen kristallinen Anteil von 70 % aus.

<u>Ausführungsbeispiel 4 (erfindungsgemäß) Sprühgranulation unter Zusatz von kristallinem Feinstaub</u>

Wässrige Na3-MGDA -Lösung mit einem Feststoffgehalt von 48,8% wurde auf einer kontinuierlich betriebenen Labor-Sprühwirbelschicht sprühgranuliert. Die konische Wirbelschicht mit einem Durchmesser unten von 150 mm und oben von 300 mm hatte innenliegende Schlauchfilter und eine pneumatische Zerstäubungsdüse, mit der von unten in die Wirbelschicht eingesprüht wurde. Die Wirbelschicht wurde mit 55 N$m^3$/h Stickstoff einer Eintrittstemperatur von 140°C und einer Wirbelschichttemperatur von 79°C betrieben. In die Wirbelschicht wurde Na3-MGDA - Sprühgranulat aus vorhergehenden Versuchen als Vorlage eingefüllt. Über einen Zeitraum von 1,92 Stunden wurde insgesamt eine Menge von 6,03 kg Lösung eingesprüht. Dazu wurde die pneumatische Zerstäubungsdüse mit 4,7 N$m^3$/h Stickstoff bei Raumtemperatur und bei einem Druck von 3,3 bar (absolut) betrieben. Feststoff wurde über eine Schnecke aus der Wirbelschicht ausgetragen, so daß die Wirbelschichthöhe konstant blieb. Der ausgetragene Feststoff wurde aller 30 Minuten ausgesiebt. Im Partikelgrößenbereich von 355 bis 1250μm lagen 46,8% der ausgetragen Partikeln. Die Schüttdichte dieser Fraktion betrug 778 kg/$m^3$ und ihr Wassergehalt betrug 11,8 Massen%. Der ausgesiebte Feinanteil kleiner 355 μm wurde aller 30 Minuten in die Wirbelschicht zurückgeführt.

Das so hergestellte Produkt war ein rieselfähiges Granulat. Die Röntgenstrukturanalyse zeigt, dass das Produkt Kristalle der vorstehend definierten ersten Modifikation enthält und 71% kristallin ist.

Das Lagerverhalten dieser Probe wurde im Exsikkator-Test bewertet. Dazu wird eine Probe von 3 g in einem offenen Wägeschälchen in einen Exsikkator bei 20°C und einer relativen Luftfeuchte von 76% über einen Zeitraum von 144 Stunden gelagert. Anschließend wird die Massezunahme der Probe ermittelt und die Rieselfähigkeit der Probe bewertet. Die Massezunahme betrug 25,8% und die Probe war nur gering verbacken und ließ sich durch leichtes Klopfen wieder in den rieselfähigen Zustand bringen.

## Patentansprüche

1. Verfahren zur Herstellung eines Pulvers enthaltend eines oder mehrere Komplexbildnersalze der allgemeinen Formel

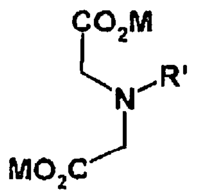

(I)

in der

R' Wasserstoff oder eine der Gruppierungen

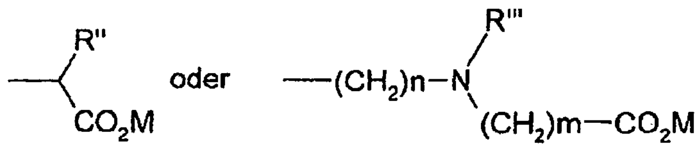

bedeutet, wobei

R'' Wasserstoff, ein $C_1$-$C_{12}$-Alkylrest oder ein -$(CH_2)_q$-COOM-Rest mit q = 1 bis 5 ist
n und m jeweils eine ganze Zahl von 0 bis 5 sind und
R''' Wasserstoff oder ein $C_1$-$C_{12}$-Alkylrest oder ein $C_2$-$C_2$-Alkenylrest ist, der zusätzlich mit bis zu 5 Hydroxylgruppen substituiert sein kann, oder eine der Gruppierungen

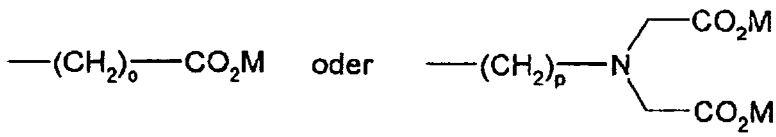

ist, in der o und p jeweils eine ganze Zahl von 0 bis 5 sind, und
M unabhängig voneinander Wasserstoff, Alkalimetall, Erdalkalimetall, Ammonium oder substituiertes Ammonium in den entsprechenden stöchiometrischen Mengen bedeutet,

- ausgehend von einer wässrigen Lösung, enthaltend das eine oder die mehreren Komplexbildnersalze in einer Konzentration von 10 bis 80 Gew.-%, bezogen auf das Gesamtgewicht der wässrigen Lösung,
- in einem Sprühtrocknungsverfahren, umfassend einen Zerstäubungsschritt und einen Trocknungsschritt,

**dadurch gekennzeichnet, dass**
der Zerstäubungsschritt unter Zusatz von kristallinem Feinstaub desselben oder derselben Komplexbildnersalze, wie sie in der wässrigen Lösung enthalten sind, oder eines oder mehrerer hiervon verschiedener Komplexbildnersalze,
mit einer Obergrenze für den mittleren Partikeldurchmesser des kristallinen Feinstaubs, die um mindestens den Faktor 2 kleiner ist als die Untergrenze des mittleren Partikeldurchmessers des nach dem Verfahren erhaltenen Pulvers,
in einem Anteil von 0,1 bis 20 Gew.-%, bezogen auf das Gewicht des nach dem Verfahren erhaltenen Pulvers, durchgeführt wird.

2. Verfahren nach Anspruch 1, **dadurch gekennzeichnet, dass** das Sprühtrocknungsverfahren in einem Sprühturm durchgeführt wird.

3. Verfahren nach Anspruch 1, **dadurch gekennzeichnet, dass** das Sprühtrocknungsverfahren eine Sprühgranulatlon ist, bei der im Zerstäubungsschritt die wässrige Lösung, enthaltend das eine oder die mehreren Komplexbildnersalze in eine Wirbelschicht eingesprüht wird, die ein Granulat des einen oder der mehreren Kompfexblldhersalze der Formel I enthält.

4. Verfahren nach Anspruch 2, **dadurch gekennzeichnet, dass** im Sprühturm eine Wirbelschicht integriert ist und eine agglomerierende Sprühtrocknung durchgeführt wird.

5. Verfahren nach Anspruch 1, **dadurch gekennzeichnet, dass** das Sprühtrocknungsverfahren eine Sprühagglomeration ist, die in einem Mischer mit bewegten Einbauten durchgeführt wird, unter Erhalt eines Agglomerats, das anschließend in einem weiteren Apparat, insbesondere in einer Wirbelschicht, fertig getrocknet wird.

6. Verfahren nach einem der Ansprüche 1 bis 5, **dadurch gekennzeichnet, dass** von einer wässrigen Lösung, die nach der entsprechenden Synthese erhalten wird, ausgegangen wird, die ca. 30 bis 50 Gew.-% des einen oder der

mehreren Komplexbildnersalze enthält, und die In einem dem Sprühtrocknungsverfahren vorgeschalteten Verfahrensschritt in einem Wärmetauscher oder einem Dünnschichtverdampfer auf ca. 55 bis 80 Gew.-% an Komplexbildnersalzen, bezogen auf das Gesamtgewicht der wässrigen Lösung, aufkonzentriert wird.

**7.** Verfahren nach einem der Ansprüche 1 bis 6, **dadurch gekennzeichnet, dass** der Trocknungsschritt bei einem Druck im Bereich von 0,1 bar absolut bis 10 bar absolut, vorzugsweise bei einem Druck im Bereich von 0,8 bar absolut bis 2 bar absolut, durchgeführt wird.

**8.** Verfahren nach einem der Ansprüche 1 bis 7, **dadurch gekennzeichnet, dass** die Verweilzeit im Trocknungaschritt im Bereich von 10 Sekunden bis zu 1 h liegt.

**9.** Formulierungen, enthaltend das gemäß einem der Ansprüche 1 bis 8 hergestellte Pulver als Komplexbildner für Erdalkali- und Schwermetallionen, in den hierfür üblichen Mengen, neben anderen üblichen Bestandteilen solcher Formulierungen.

**10.** Formulierungen nach Anspruch 9, **dadurch gekennzeichnet, dass** es sich um Wasch- und Reinigungsmittelformullerungen handelt.

**11.** Verwendung des nach einem Verfahren nach einem der Ansprüche 1 bis 8 hergestellten Pulvers zur Herstellung von Pressagglomeraten.

**12.** Verwendung der Pressagglomerate nach Anspruch 11 zum Einsatz in festen Reinigungsmitteln.

**13.** Verwendung nach Anspruch 12, **dadurch gekennzeichnet, dass** die festen Reinigungsmittel zum Einsatz in Geschirrspülmaschinen bestimmt sind.

**14.** Verwendung nach Anspruch 13, **dadurch gekennzeichnet, dass** die Reinigungsmittel für automatische Geschirrspülmaschinen In Form von Tabs vorliegen.

## Claims

**1.** A process for the preparation of a powder comprising one or more complexing agent salts of the general formula

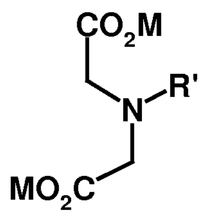

(I)

in which

R' is hydrogen or one of the groups

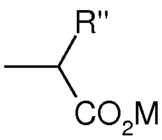

or

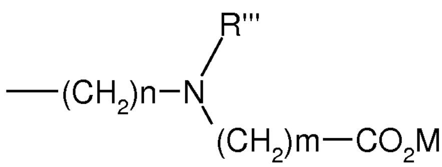

where

R'' is hydrogen, a $C_1$-$C_{12}$-alkyl radical or a $-(CH_2)_q$-COOM radical where q = 1 to 5
n and m are in each case an integer from 0 to 5 and
R''' is hydrogen or a $C_1$-$C_{12}$-alkyl radical or a $C_2$-$C_{12}$-alkenyl radical which may be additionally substituted by

up to 5 hydroxyl groups, or one of the groups

$$-(CH_2)_o-CO_2M$$

or

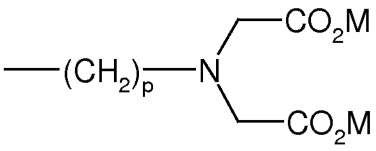

in which o and p are in each case an integer from 0 to 5, and
M, independently of the others, is hydrogen, alkali metal, alkaline earth metal, ammonium or substituted ammonium in the corresponding stoichiometric amounts,

- starting from an aqueous solution comprising the one or more complexing agent salts in a concentration of from 10 to 80% by weight, based on the total weight of the aqueous solution,
- in a spray-drying process, comprising an atomization step and a drying step,

wherein
the atomization step is carried out with the addition of crystalline fine dust of the same complexing agent salt(s) as are present in the aqueous solution, or one or more complexing agent salts different therefrom,
with an upper limit for the average particle diameter of the crystalline fine dust which is lower by at least a factor of 2 than the lower limit of the average particle diameter of the powder obtained after the process,
in a fraction of from 0.1 to 20% by weight, based on the weight of the powder obtained after the process.

**2.** The process according to claim 1, wherein the spray-drying process is carried out in a spray-tower.

**3.** The process according to claim 1, wherein the spray-drying process is a spray-granulation in which, in the atomization step, the aqueous solution comprising the one or more complexing agent salts is sprayed into a fluidized bed which comprises granules of the one or more complexing agent salts of the formula I.

**4.** The process according to claim 2, wherein, in the spray-tower, a fluidized bed is integrated and an agglomerating spray-drying is carried out.

**5.** The process according to claim 1, wherein the spray-drying process is a spray-agglomeration which is carried out in a mixer with agitated internals, to give an agglomerate which is then fully dried in a further apparatus, in particular in a fluidized bed.

**6.** The process according to any one of claims 1 to 5, wherein the starting material used is an aqueous solution which is obtained by the corresponding synthesis and which comprises ca. 30 to 50% by weight of the one or more complexing agent salts, and which is concentrated in a process step connected upstream of the spray-drying process in a heat exchanger or a thin-film evaporator to ca. 55 to 80% by weight of complexing agent salts, based on the total weight of the aqueous solution.

**7.** The process according to any one of claims 1 to 6, wherein the drying step is carried out at a pressure in the range from 0.1 bar absolute to 10 bar absolute, preferably at a pressure in the range from 0.8 bar absolute to 2 bar absolute.

**8.** The process according to any one of claims 1 to 7, wherein the residence time in the drying step is in the range from 10 seconds up to 1 h.

**9.** A formulation comprising the powder prepared according to any one of claims 1 to 8 as complexing agent for alkaline earth metal ions and heavy metal ions in the amounts customary for this purpose, besides other customary constituents of such formulations.

**10.** The formulation according to claim 9, which is a detergent or cleaner formulation.

**11.** The use of the powder prepared by a process according to any one of claims 1 to 8 for producing compression agglomerates.

**12.** The use of the compression agglomerates according to claim 11 for use in solid cleaners.

**13.** The use according to claim 12, wherein the solid cleaners are intended for use in dishwashers.

**14.** The use according to claim 13, wherein the cleaners for automatic dishwashers are in the form of tablets.

**Revendications**

**1.** Procédé pour la production d'une poudre contenant un ou plusieurs sels complexants de formule générale

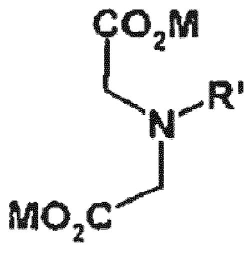

(I)

dans laquelle

R' représente un atome d'hydrogène ou l'un des groupements

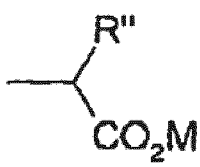

ou

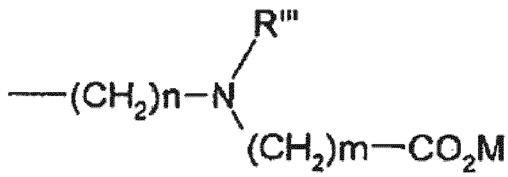

où

R" représente un atome d'hydrogène, un radical alkyle en $C_1$-$C_{12}$ ou un radical - $(CH_2)_q$-COOM où q vaut de 1 à 5
n et m représentent chacun un nombre entier valant de 0 à 5 et
R"' est un atome d'hydrogène ou un radical alkyle en $C_1$-$C_{12}$ ou un radical alcényle en $C_2$-$C_{12}$, qui peut en plus être substitué par jusqu'à 5 groupes hydroxy,
ou est l'un des groupements

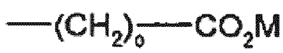

ou

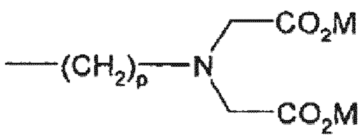

dans lesquels o et p représentent chacun un nombre entier valant de 0 à 5, et
M représente chaque fois indépendamment un atome d'hydrogène, un métal alcalin, un métal alcalino-terreux, l'ion ammonium ou un ion ammonium substitué,
en les quantités stoechiométriques correspondantes,

- à partir d'une solution aqueuse contenant ledit un ou lesdits plusieurs sels complexants à une concentration de 10 à 80 % en poids, par rapport au poids total de la solution aqueuse,
- dans un procédé de séchage par atomisation, comprenant une étape de pulvérisation et une étape de séchage,

**caractérisé en ce**
**qu'**on effectue l'étape de pulvérisation avec addition de poudre fine cristalline du même ou des mêmes sels complexants, tels qu'ils sont contenus dans la solution aqueuse, ou d'un ou de plusieurs sels complexants différents de ceux-ci,

avec une limite supérieure du diamètre moyen de particule de la poudre fine cristalline qui est inférieure par au moins le facteur 2 à la limite inférieure du diamètre moyen de particule de la poudre obtenue selon le procédé, en une proportion de 0,1 à 20 % en poids, par rapport au poids de la poudre obtenue selon le procédé.

**2.** Procédé selon la revendication 1, **caractérisé en ce que** le procédé de séchage par atomisation est effectué dans une tour de pulvérisation.

**3.** Procédé selon la revendication 1, **caractérisé en ce que** le procédé de séchage par atomisation est une granulation par pulvérisation, dans laquelle dans l'étape de pulvérisation la solution aqueuse, contenant ledit un ou lesdits plusieurs sels complexants, est pulvérisée dans une couche tourbillonnaire qui contient un granulé dudit un ou desdits plusieurs sels complexants de formule I.

**4.** Procédé selon la revendication 2, **caractérisé en ce que** dans la tour de pulvérisation est intégrée une couche tourbillonnaire et est effectué un séchage par atomisation agglomérant.

**5.** Procédé selon la revendication 1, **caractérisé en ce que** le procédé de séchage par atomisation est une agglomération par pulvérisation qui est effectuée dans un mélangeur à inserts mobiles, avec obtention d'un agglomérat, qui est ensuite soumis à un séchage de finition dans un autre appareil, en particulier dans une couche tourbillonnaire.

**6.** Procédé selon l'une quelconque des revendications 1 à 5, **caractérisé en ce qu'**on part d'une solution aqueuse, qui est obtenue après la synthèse correspondante, qui contient environ 30 à 50 % en poids dudit un ou desdits plusieurs sels complexants, et qui dans une étape de processus raccordée en amont au processus de séchage par atomisation est concentrée jusqu'à environ 55 à 80 % en poids des sels complexants, par rapport au poids total de la solution aqueuse, dans un échangeur thermique ou un évaporateur à couche mince.

**7.** Procédé selon l'une quelconque des revendications 1 à 6, **caractérisé en ce qu'**on effectue l'étape de séchage sous une pression dans la plage de 0,1 bar (absolue) à 10 bars (absolue), de préférence sous une pression dans la plage de 0,8 bar (absolue) à 2 bars (absolue).

**8.** Procédé selon l'une quelconque des revendications 1 à 7, **caractérisé en ce que** le temps de séjour dans l'étape de séchage se situe dans la plage allant de 10 secondes à 1 h.

**9.** Compositions, contenant la poudre produite selon l'une quelconque des revendications 1 à 8, en tant que complexant pour des ions de métaux alcalino-terreux et de métaux lourds, en les quantités usuelles à cette fin, en plus d'autres composants usuels de telles compositions.

**10.** Compositions selon la revendication 9, **caractérisées en ce qu'**il s'agit de compositions de produits de lavage et de nettoyage.

**11.** Utilisation de la poudre produite conformément à un procédé selon l'une quelconque des revendications 1 à 8, pour la fabrication d'agglomérats pressés.

**12.** Utilisation des agglomérats pressés selon la revendication 11, pour l'utilisation dans des produits de nettoyage solides.

**13.** Utilisation selon la revendication 12, **caractérisée en ce que** les produits de nettoyage solides sont destinés à l'emploi dans des machines à laver la vaisselle.

**14.** Utilisation selon la revendication 13, **caractérisée en ce que** les produits de nettoyage pour machines à laver la vaisselle automatiques se trouvent sous forme de tablettes.

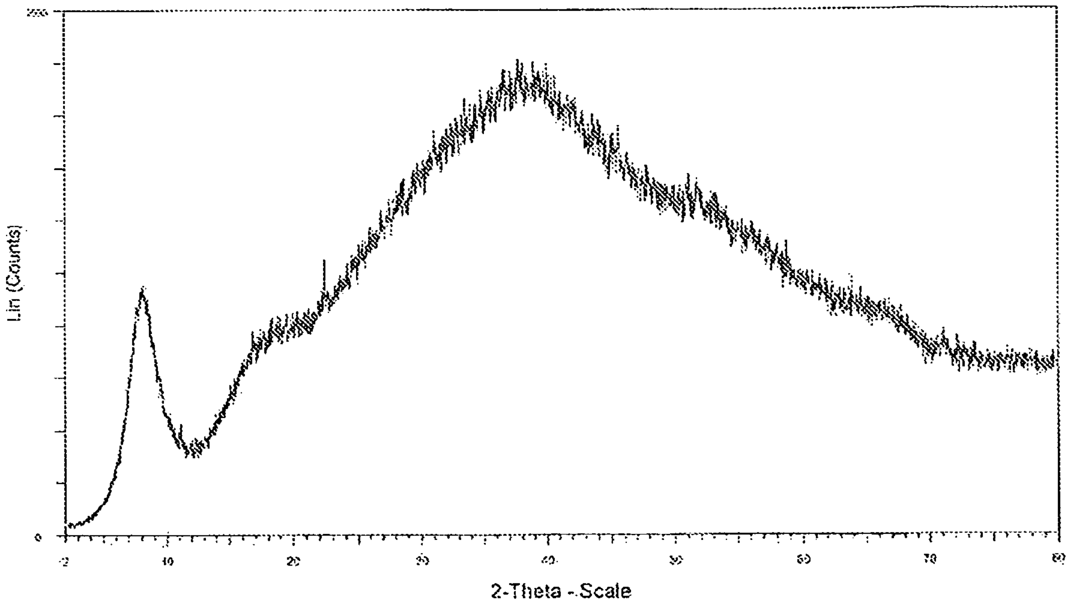


FIG. 1

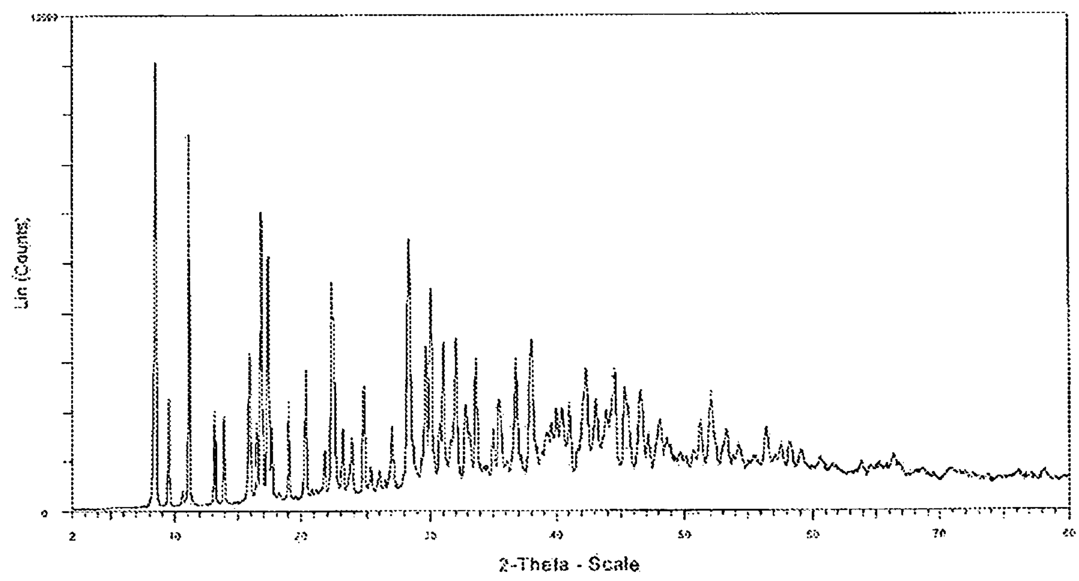


FIG. 2

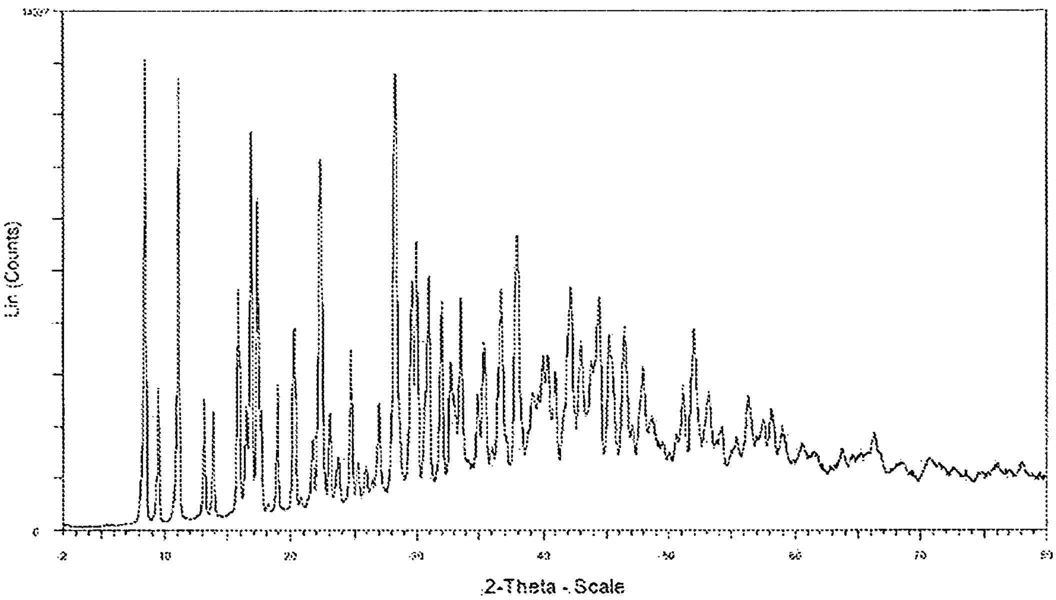


FIG. 3

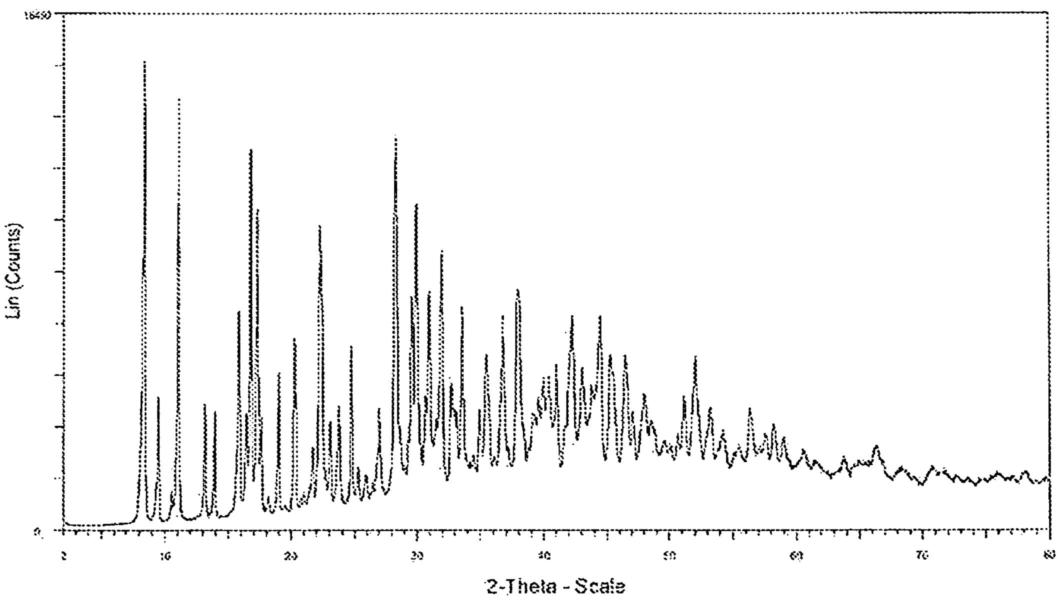


FIG. 4

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 94029421 A
- US 5849950 A
- WO 2006120129 A
- EP 0845456 A